# EUROPEAN PATENT SPECIFICATION

(11) **EP 1 121 074 B1**
(45) Date of publication and mention of the grant of the patent: **23.07.2008**
(21) Application number: 99949230.9
(22) Date of filing: 14.10.1999
(51) Int. Cl.: A61F 2/38

(54) **JELLYBEAN KNEE JOINT (IMPROVEMENT)**
OBERFLÄCHENOPTIMIERTE KNIEGELENKFLÄCHEN
PROTHESE DE GENOU AMELIOREE

(30) Priority: 16.10.1998 GB 9822711; 21.01.1999 GB 9901331
(43) Date of publication of application: 08.08.2001
(73) Proprietor: Walker, Peter Stanley, Moor Park, Middlesex HA6 2NP (GB)
(72) Inventor: Walker, Peter Stanley, Moor Park, Middlesex HA6 2NP (GB)
(74) Representative: Woodcraft, David Charles
(86) International application number: PCT/GB1999/003407
(87) International publication number: WO 2000/023011

(56) References cited:
- EP-A- 0 567 705
- EP-A- 0 634 155
- WO-A-98/46171
- US-A- 4 770 663

## Description

This invention relates to prosthetic joints, especially total knee replacement prostheses.

In the natural knee, the femoral-tibial contact point is displaced from anterior to posterior as the knee is flexed from zero to maximum. This effect is more pronounced on the lateral side than the medial. Such motion results in effective lever arms for the quadriceps and hamstrings at appropriate phases in the movement of the joint and facilitates a high range of flexion. Ideally, a total knee replacement prosthesis should mimic such displacements. In practice, for existing designs this is not the case. For example, existing posterior cruciate ligament (PCL) sacrificing designs tend to be excessively constrained, where the femur locates close to the bottom of the tibial dish throughout flexion. PCL-preserving designs tend to show variable and unpredictable motion patterns, mainly because the femoral-tibial surfaces are less constrained and the PCL tension varies between knees.

One approach to controlling the motion is to provide cam surfaces located in the intercondylar notch which interact at the appropriate stage in flexion to cause the femoral component to roll back. However, cams have certain disadvantages. There can be an impact when they engage, which may contribute to progressive wear and deformation. This effect is increased if internal-external rotation occurs simultaneously. The anterior-posterior translations are opposed to the natural tendency of the femur to locate at the bottom of the tibial dish, thus increasing the contact forces. Most cams are designed to produce roll-back : with flexion, but not roll-forward with extension.

The present invention is based on a novel concept of gradually changing the frontal profile of the femoral condylar bearing surfaces from the distal to the posterior region, in such a way as to cause the femoral component. by interaction with a similarly changing profile in the tibial bearing surfaces, to displace posteriorly with flexion and anteriorly with extension. The concept is based on the femoral bearing surfaces locating at the lowest point on the tibial bearing surfaces or finding the positions of at least potential energy, at each angle of flexion.

The reader may be further enlightened as to the state of the art by reference to EP-A-0567705, which is considered to be the closest prior art and by reference to which the present invention is characterised. EP-A-0567705 discloses a total knee replacement prosthesis having an abutment piece comprising an anterior head extended rearwards by a body of toroidal shape and situated in the intercondylar space of the femoral element. A corresponding cavity is situated on a median projection of the tibial element. In the flexed position, the abutment piece bears forwards in the cavity in order to counter a posterior dislocation of the joint. The sagittal and transverse curvatures of the abutment piece are greater than those of the cavity. The abutment piece can therefore roll in the cavity without impeding the movements of flexion and rotation of the knee.

According to the present invention there is provided a condylar total knee replacement prosthesis (TKR) which comprises: a femoral component having a pair of condylar-bearing surfaces and a tibial component having dished tibial bearing surfaces for receiving said condylar bearing surfaces, characterised in that, each condylar surface when viewed as a series of sagittal slices has a curvature such that the contact point of each slice with the corresponding portion of the tibial-bearing surface is anterior to one adjacent slice, and posterior to the other.

Thus the present invention provides a condylar total knee replacement prosthesis (TKR) which comprises a femoral component having a pair of condylar-bearing surfaces and a tibial component having dished tibial bearing surfaces for receiving said condylar bearing surfaces, wherein the frontal profiles of the bearing surfaces are designed so that the contacts points move outwardly, i.e. medially and laterally, as the knee undergoes increasing degrees of flexion.. As a result, when viewed from above, the dished areas representing the contact points diverge from anterior to posterior. The effect of this is that each condylar surface when viewed as

a series of sagittal slices has a curvature such that the contact point of each slice with the corresponding portion of the tibial-bearing surface is anterior to one adjacent slice, and posterior to the other. By constructing the profiles of the bearing surfaces as though they were a very large number of thin sagittal slices, the overall bearing surfaces will be smooth and free from any sharp discontinuities. Due to these surfaces, the contact points at the lowest points will move posteriorly with flexion and anterior with extension.

As will be described in more detail subsequently in this specification, it is also possible to shape the contacting femoral and tibial bearing surfaces so that the points of contact, when viewed from above, converge as the joint moves from extension to flexion.

An additional inducement to the motion is produced by the intercondylar region. Due to the diverging femoral condyles, the intercondylar groove becomes deeper with flexion. This produces an intercondylar hump between the dished receiving areas on the tibial surface and the height of the hump increases when seen in a frontal plane from anterior to posterior. Because of this. as the knee begins to extend from flexion, the femoral component rolls down the hump and is caused to slide forward.

A fuller understanding of the invention will be gained from the following detailed description and accompanying drawings, in which:
Figure 1 is a diagrammatic sagittal view of a TKR prosthesis in accordance with the invention;

Figure 2 is a plan view of a tibial component showing the divergent pattern of the dished contact areas with the condylar bearing surfaces;
Figures 3 (a) to 3 (c) are anterior views of the femoral component at the indicated degrees of flexion;

Figures 4 (a) to 4 (c) are respectively perspective, anterior and posterior views of the tibial bearing component;

Figure 5 is a diagrammatic representation of a further embodiment in which the frontal radius of the femoral component is seen to decrease with the flexion angle of the component;

Figure 6 is a plan view of a femoral component showing the natural convergence of the component from posterior to anterior;

Figure 7 (a) is a sagittal view; and

Figure 7 (b) is a frontal/upper view of a further embodiment in accordance with the invention, in which contact points converge from extension to high flexion.

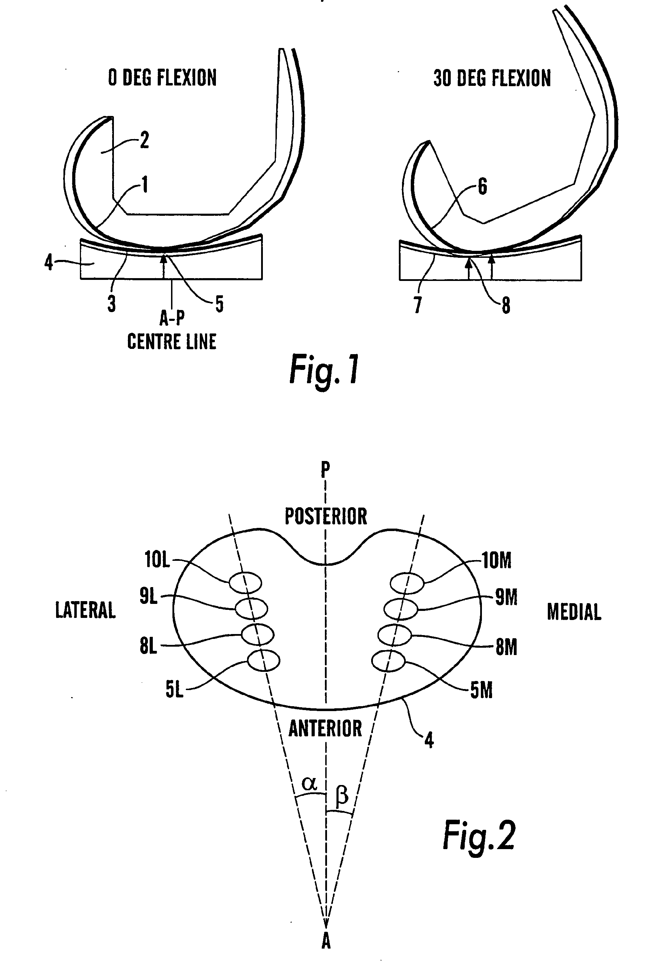

Referring to Figures 1 and 2 of the drawings, the condylar and tibial bearing surfaces can be visualised as formed from a series of sagittal slices, each slice being a different-distance from the A-P centreline. Each of the slices. (only two of which are shown in Figure 1), has a profile which ensures that its contact point or area with the tibial surface is different. In Figure 1, a first slice 1 of the femoral condyle 2 contacts slice 3 of the tibial condyle 4 at contact point 5. The tibial component in this embodiment may be a shaped plastics member fixed in a conventional way to a tibial base plate. This contact point 5 is also shown in Figure 2 as 5L (lateral) and 5M (medial), which represents the position at zero flexion. In Figure 1, a second sagittal slice 6 contacts slice 7 of the tibial condyle at contact point 8. when the prosthesis is at say 30 degrees of flexion. This contact point 8 is more posterior to the A-P centreline than contact point 5. The profile of the bearing surfaces can be varied by changing the radius in different slices, or changing the centre of the radii. or by a combination of both measures.

As can be seen from Figure 2, the contact points for other sagittal slices 9L, 9M and 10L and 10M for 90 and 120 flexion are progressively more posterior to the A-P centreline 'L' and 'M' indicate that the points are on the lateral or medial side respectively of the prosthesis and it will be seen that the contact points also diverge from anterior to posterior. Such divergence may be equal or different, e. g. the angles a and P may be equal or different. It will be appreciated that there will be slices giving rise to contact points between those shown in Figure 2 and that the profiles of the slices will preferably blend smoothly into each other.

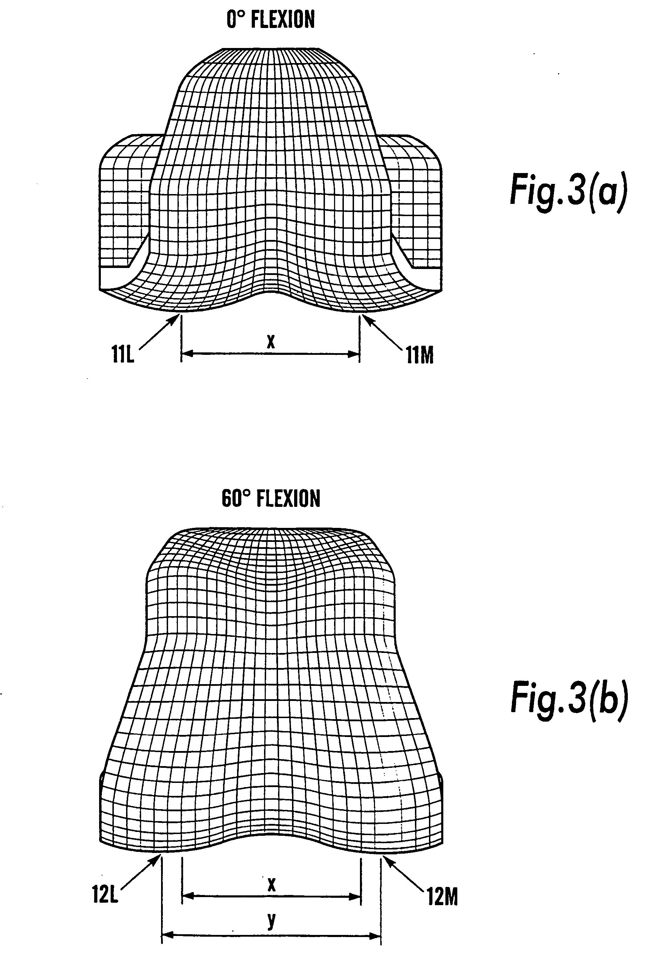

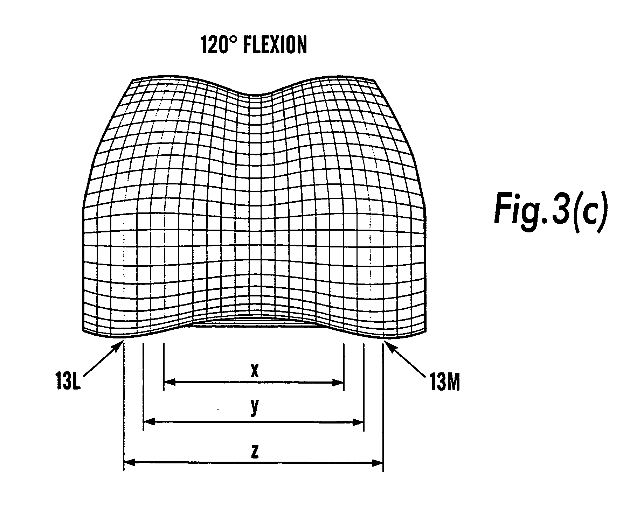

Figures 3 (a)-3 (c) are frontal plane views of the femoral component at 0 , 60 and 120 flexion respectively. It will be seen that the points 11 L and 11 M in Figure 3 (a), which are designed to make contact with corresponding points on the tibial bearing surface, generally lie on a vertical plane which passes generally centrally through each condyle. However, this is not essential and the contact points at 0 flexion may be closer together. The distance separating contact points 11 L and 11 M is indicated as dimension 'x'. At 60 flexion, the condylar surfaces are shaped so that the contact points 12L and 12M are separated by a distance 'y' which is greater than dimension 'x'. When the prosthesis reaches maximum flexion, e. g. at 120 , the contact points 13L and 13M are still further separated by a distance 'z' as indicated in Figure 3 (c).

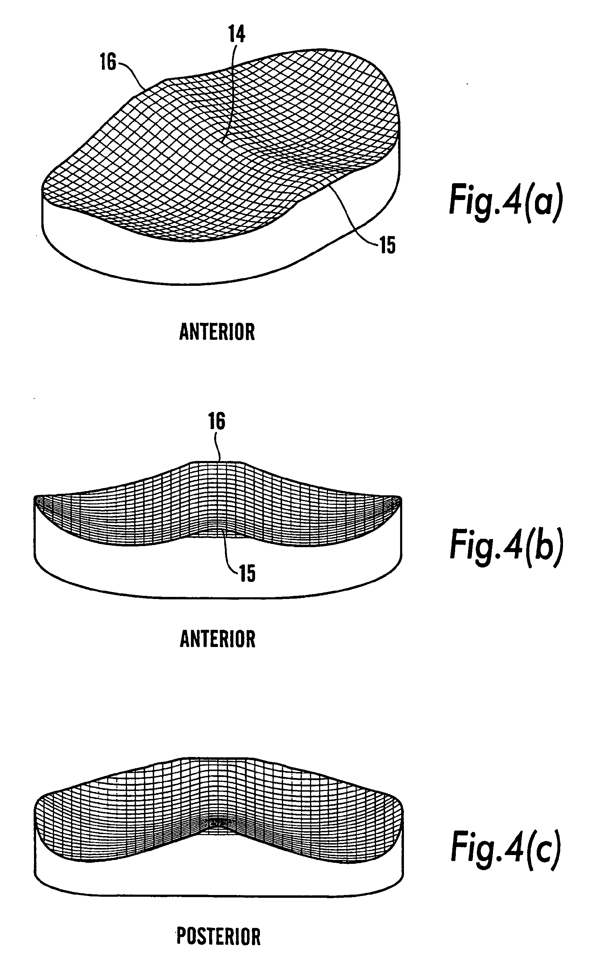

The tibial bearing surface is shown in Figures 4 (a) to 4 (c). It will be seen that the width of the dished receiving portion increases from anterior to posterior, that is, moving outwardly, laterally and medially. The interaction of the diverging femoral surfaces and diverging tibial surfaces will cause the contact points to move steadily posterior with flexion and anterior with extension. The thickness of the plastics meniscal member is generally constant at the lowest points of the sagittal slices. Also, an intercondylar hump 14 extends in an A-P direction and is lower at the anterior side 15 than at the posterior side 16. From anterior to posterior the hump rises, the rise being steepest on approaching the posterior side. As mentioned above, this sloping ridge has the effect of assisting roll forward movement as the pivoting movement of the knee changes from the fully flexed position towards extension.

Figure 2 shows the dished contact points on the medial and lateral sides. In figure 2, the tibial contact points on the lateral and medial sides are symmetric.

However there are advantages in making the contact point locations on the medial side more closely spaced than on the lateral. This would have the effect of causing the femoral component to rotate about a vertical axis through the tibia as the knee was flexed. Hence the femoral component can be influenced to rotate as it flexes, thereby replicating more closely the movement of the natural knee.

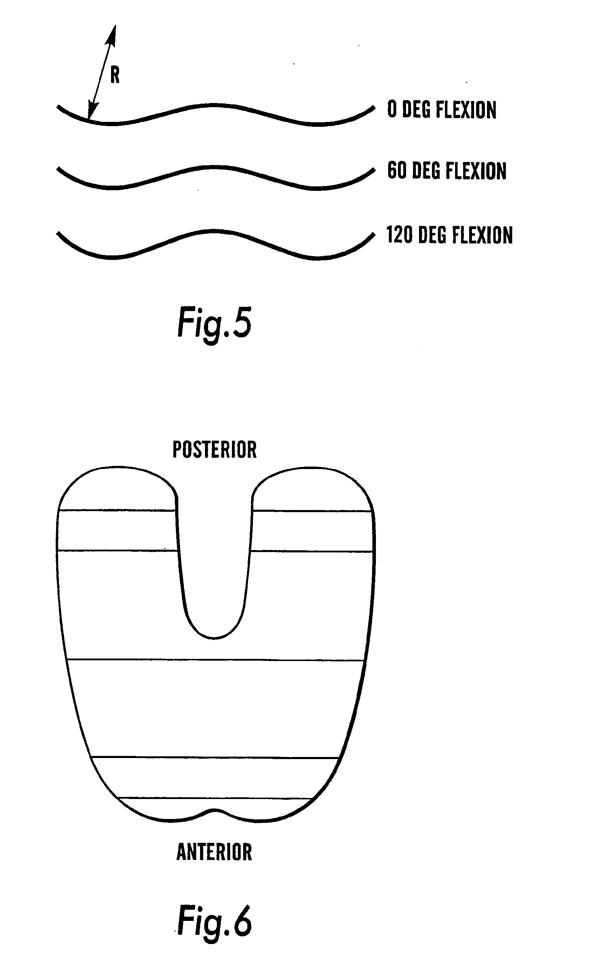

Further means of guiding the anterior-posterior motion is to change the frontal profile of the femoral surfaces. Figure 5 shows the frontal radius R decreasing with the flexion angle of the component. Yet further means of guiding the anterior posterior motion is to make use of the natural convergence of the femoral component from posterior to anterior (Figure 6), such that it reacts with corresponding convergence on the tibial surface.

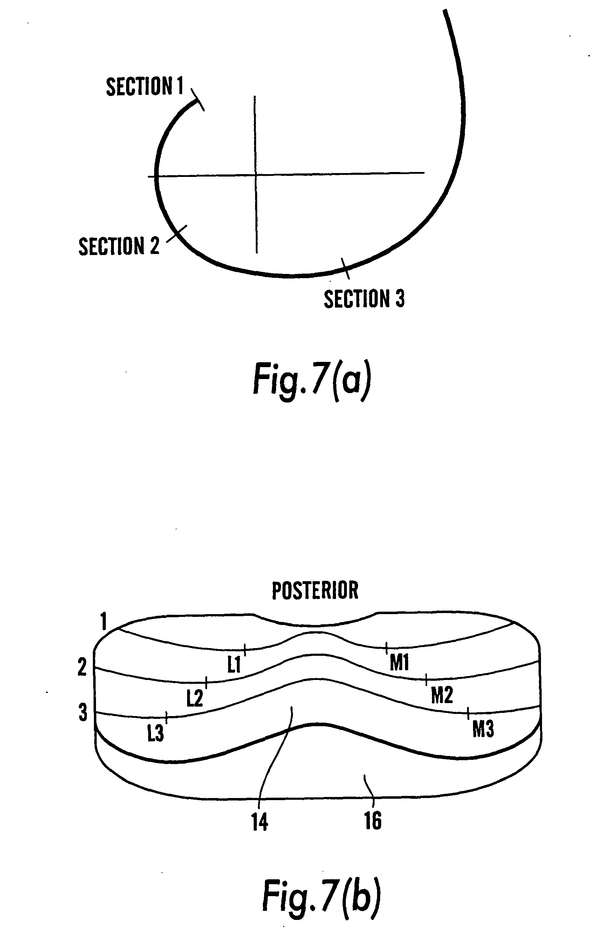

It will also be appreciated that rather than the bearing surfaces diverging from anterior to posterior, the principle works equally well if the surfaces converge with flexion. Similarly, the frontal radii can increase rather than decrease with flexion angle. The former embodiment is illustrated diagrammatically in Figures 7 (a) and 7 (b).

Figure 7 (a) is a sagittal view of the femoral surface and Figure 7 (b) is a frontal/upper view of the tibial surface. As indicated in Figure 7 (a), the femoral component has 3 sections located such that section 3 will be in contact with the tibial surface in the region of full extension, section 2 will contact in the mid range and section 1 will be in contact in high flexion.

The tibial and femoral bearing surfaces are formed so that the areas of contact between the two components converge from extension to flexion. Figure 7 (b) shows by reference numerals 1,2 and 3 the areas in which femoral view section 1,2 or 3 of the femoral component will be in contact as the prosthesis pivots from extension to full flexion. It will be clear that during this movement, the contact areas will move inwardly laterally and medially. Figure 7 (b) shows the shape of the tibial surface..

The shape of the corresponding femoral and tibial sections is similar but with a small clearance. In Figure 7 (b) the short vertical lines at each section 1,2 or 3 show the bearing spacing which represents the lowest points in each section. It will be understood that when the knee is in extension, the lowest points of the femoral and tibial components will align along section 3 at points L3 and M3. When the knee is in mid-flexion, the alignment will be along section 2 at contact points L2 and M2. Thus, there will be a posterior displacement of the contact points. Similarly, as the knee flexes further to section 1 there will be a further posterior displacement at high flexion to contact points Ll and Ml.

In contrast with the arrangement shown in Figures 4 (a) to 4 (c), the intercondylar hump 14 becomes narrower as the contact points move steadily posteriorly with flexion. Also, the intercondylar hump in the embodiment of Figure 7 (b) is higher at the anterior side 16 than at the posterior. This posteriorly directed slope assists roll back as the knee changes from extension towards high flexion.

A knee prosthesis having converging contact points has several potential advantages. For example, there will be better resistance to varus-valgus moments in early stages of flexion because of the wider contact base. In high flexion, there will be reduced torque because of the narrow contact base, thereby allowing greater or easier rotational freedom. Also, the intercondylar region of the femoral surface which interacts with the patella will be broader and deeper. A possible disadvantage is that the sides of the femoral component in the anterior and superior region may need to be more prominent.

In an analogous way to the'divergent' embodiment described in Figures 4 (a) to 4 (c), the 'convergent' embodiment of Figures 7 (a) and 7 (b) can be designed to rotate as it flexes, e. g. by positioning the contact points in the sequential sections so that the knee tends to rotate medially as it flexes.

Although the design of the interacting bearing surfaces in accordance with the invention is intended to largely replace the use of intercondylar cams, it may also be advantageous to provide for the use of cams in the intercondylar region, in addition to the shaped bearing surfaces, if a particularly positive guide to The motion over a part of the flexion-extension range is required. Examples of intercondylar cams are described in WO 98/46171, US 4209861 and EPA 0627203.

In the above description, it has been assumed for greater simplicity that the tibial bearing component is fixed. However, mobile bearing components may be employed where the plastic tibial bearing component is allowed to rotate on a smooth metal base plate and thus enables greater amounts of rotational movement to be achieved. Examples of tibial components in which a plastics meniscal member is rotatably supported in a metal base plate are shown in EPA 0636353 and 0627203.

## Claims

1. A condylar total knee replacement prosthesis (TKR) which comprises:
a femoral component having a pair of condylar-bearing surfaces and
a tibial component having dished tibial bearing surfaces for receiving said condylar bearing surfaces, **characterised in that**,
each condylar surface when viewed as a series of sagittal slices has a curvature such that the contact point (5, 8, 9, 10) of each slice with the corresponding portion of the tibial-bearing surface is anterior to one adjacent slice, and posterior to the other.

2. A TKR prosthesis as claimed in claim 1 wherein the contact points (5, 8, 9, 10) of said sagittal slices with the corresponding portions of the tibial-bearing surface, when viewed from above the tibial-bearing surface, generally diverge from anterior to posterior.

3. A TKR prosthesis as claimed in claim 1 wherein the contact points (5, 8, 9, 10) of said sagittal slices with the corresponding portions of the tibial bearing surface, when viewed from above the tibial bearing surface, generally converge from anterior to posterior.

4. A TKR prosthesis as claimed in claim 2 wherein the contact points of one condylar bearing surface with its corresponding tibial bearing surface move posteriorly at a different rate compared with the contact points of the other condylar bearing surface, thereby causing the femur to pivot axially of the tibia as the knee undergoes flexion.

5. A TKR prosthesis as claimed in claim 3 wherein the contact points on the lateral side move posteriorly at a greater rate than the contact points on the medial side.

6. A TKR prosthesis as claimed in claim 4 wherein the contact points follow the natural convergence of the femoral component from posterior to anterior.

7. A TKR prosthesis as claimed in any one of the preceding claims wherein an intercondylar hump (14) is formed in the tibial bearing component, which changes in height with respect to the tibial contact points in a posterior direction.

8. A TKR prosthesis according to any one of the preceding claims, wherein the frontal radii (R) of the femoral surface at points corresponding to different degrees of flexion changes with the flexion angle of the component.

9. A TKR prosthesis as claimed in any one of the preceding claims wherein the shape of the femoral condylar and tibial-bearing surfaces changes smoothly across the width of the knee.

10. A TKR prosthesis as claimed in any one of the proceeding claims wherein the femoral condylar and tibial-bearing surfaces are shaped in the intercondylar region to provide interacting cam surfaces which assist in controlling relative movement between the femoral and tibial components during flexion and/or extension.

11. A TKR prosthesis as claimed in any one of the proceeding claims wherein the tibial component comprises a meniscal component rotatably supported on a tibial platform.

12. A TKR according to any one of the preceding claims which comprises:
tibial bearing surfaces so shaped that when viewed as a series of transverse slices extending transversely to the anterior-posterior direction, contact points (5, 8, 9, 10) between the bearing surfaces are displaced posteriorly with flexion and anteriorly with extension or vice versa, and each contact point (5, 8, 9, 10) constitutes the lowest point on the tibial bearing surface at a given angle of flexion.

## Patentansprüche

1. Kondyläre Knietotalersatzprothese (TKR), die umfasst:
eine femorale Komponente mit einem Paar kondylärer Lagerflächen und eine tibiale Komponente mit konkaven tibialen Lagerflächen zur Aufnahme dieser kondylären Lagerflächen, **dadurch gekennzeichnet, dass**,
jede kondyläre Fläche, wenn sie als eine Reihe sagittaler Schnitte betrachtet wird, eine solche Krümmung aufweist, dass sich der Kontaktpunkt (5, 8, 9, 10) jedes Schnitts mit dem entsprechenden Teil der tibialen Lagerfläche anterior zu einem benachbarten Schnitt und posterior zum anderen befindet.

2. TKR-Prothese nach Anspruch 1, wobei die Kontaktpunkte (5, 8, 9, 10) der sagittalen Schnitte mit den entsprechenden Teilen der tibialen Lagerfläche, wenn sie in Draufsicht auf die tibiale Lagerfläche betrachtet werden, im allgemeinen von anterior nach posterior divergieren.

3. TKR-Prothese nach Anspruch 1, wobei die Kontaktpunkte (5, 8, 9, 10) der sagittalen Schnitte mit den entsprechenden Teilen der tibialen Lagerfläche, wenn sie in Draufsicht auf die tibiale Lagerfläche betrachtet werden, im allgemeinen von anterior nach posterior konvergieren.

4. TKR-Prothese nach Anspruch 2, wobei die Kontaktpunkte einer kondylären Lagerfläche mit ihrer entsprechenden tibialen Lagerfläche mit einer anderen Geschwindigkeit nach posterior wandern als die Kontaktpunkte der anderen kondylären Lagerfläche, wodurch eine axiale Drehung des Femurs um die Tibia bewirkt wird, während das Knie eine Beugung erfährt.

5. TKR-Prothese nach Anspruch 3, wobei die Kontaktpunkte auf der lateralen Seite mit einer größeren Geschwindigkeit nach posterior wandern als die Kontaktpunkte auf der medialen Seite.

6. TKR-Prothese nach Anspruch 4, wobei die Kontaktpunkte der natürlichen Konvergenz der femoralen Komponente von posterior nach anterior folgen.

7. TKR-Prothese nach einem der vorhergehenden Ansprüche, wobei ein interkondylärer Höcker (14) in der tibialen Lagerkomponente ausgebildet ist, der sich in posteriorer Richtung in Bezug auf die tibialen Kontaktpunkte in der Höhe ändert.

8. TKR-Prothese nach einem der vorhergehenden Ansprüche, wobei sich die frontalen Radien (R) der femoralen Fläche an Punkten, die verschiedenen Beugungsgraden entsprechen, mit dem Beugungswinkel der Komponente ändern.

9. TKR-Prothese nach einem der vorhergehenden Ansprüche, wobei sich die Form der femoralen kondylären und tibialen Lagerflächen über die Breite des Knies stufenlos verändert.

10. TKR-Prothese nach einem der vorhergehenden Ansprüche, wobei die femoralen kondylären and tibialen Lagerflächen im interkondylären Bereich so gestaltet sind, dass sie zusammenwirkende Nockenflächen bieten, welche helfen, die Relativbewegung zwischen der femoralen und der tibialen Komponente während der Beugung und/oder Streckung zu kontrollieren.

11. TKR-Prothese nach einem der vorhergehenden Ansprüche, wobei die tibiale Komponente eine meniskale Komponente umfasst, die drehbar auf einer tibialen Plattform gelagert ist.

12. TKR nach einem der vorhergehenden Ansprüche, die umfasst:
tibiale Lagerflächen, welche so geformt sind, dass, wenn sie als ein Reihe von Querschnitten betrachtet werden, die sich quer zur anterior-posterioren Richtung erstrecken, Kontaktpunkte (5, 8, 9, 10) zwischen den Lagerflächen bei Beugung nach posterior und bei Streckung nach anterior verschoben werden oder umgekehrt und jeder Kontaktpunkt (5, 8, 9, 10) bei einem gegebenen Beugungswinkel den tiefsten Punkt auf der tibialen Lagerfläche bildet.

## Revendications

1. Prothèse totale de genou de type condylien (TKR) comprenant :
un composant fémoral ayant une paire de surfaces d'appui condyliennes, et
un composant tibial ayant des surfaces d'appui tibiales en forme de plateau pour recevoir lesdites surfaces d'appui condyliennes, **caractérisée en ce que** :
chaque surface condylienne lorsqu'elle est observée comme une série de vues anatomiques sagittales a une courbure telle que le point de contact (5, 8, 9, 10) de chaque vue anatomique avec la partie correspondante de la surface d'appui tibiale est antérieur à la vue anatomique adjacente, et postérieur à l'autre.

2. Prothèse TKR selon la revendication 1, dans laquelle les points de contact (5, 8, 9, 10) desdites vues anatomiques sagittales avec les parties correspondantes de la surface d'appui tibiale, lorsqu'elles sont observées au-dessus de la surface d'appui tibiale, divergent généralement d'avant en arrière.

3. Prothèse TKR selon la revendication 1, dans laquelle les points de contact (5, 8, 9, 10) desdites vues anatomiques sagittales avec les parties correspondantes de la surface d'appui tibiale, lorsqu'elles sont observées au-dessus de la surface d'appui tibiale, convergent généralement d'avant en arrière.

4. Prothèse TKR selon la revendication 2, dans laquelle des points de contact d'une surface d'appui condylienne avec sa surface d'appui tibiale correspondante se déplacent de manière postérieure à une vitesse différente par rapport aux points de contact de l'autre surface d'appui condylienne, amenant ainsi le fémur à pivoter de manière axiale par rapport au tibia lorsque le genou subit une flexion.

5. Prothèse TKR selon la revendication 3, dans laquelle les points de contact sur le côté latéral se déplacent en arrière à une plus grande vitesse que les points de contact sur le côté médian.

6. Prothèse TKR selon la revendication 4, dans laquelle les points de contact suivent la convergence naturelle du composant fémoral d'arrière en avant.

7. Prothèse TKR selon l'une quelconque des revendications précédentes, dans laquelle une bosse bicondylienne (14) est formée dans le composant d'appui tibial, qui change en hauteur par rapport aux points de contact tibiaux dans une direction postérieure.

8. Prothèse TKR selon l'une quelconque des revendications précédentes, dans laquelle les rayons frontaux (R) de la surface fémorale aux points correspondant à différents degrés de flexion changent avec l'angle de flexion du composant.

9. Prothèse TKR selon l'une quelconque des revendications précédentes, dans laquelle la forme des surfaces d'appui tibiale et condylienne fémorales change progressivement sur la largeur du genou.

10. Prothèse TKR selon l'une quelconque des revendications précédentes, dans laquelle les surfaces d'appui tibiale et condylienne fémorales sont formées dans la région bicondylienne pour fournir des surfaces de came d'action mutuelle qui aident à contrôler le mouvement relatif entre les composants fémoral et tibial pendant la flexion et/ou l'extension.

11. Prothèse TKR selon l'une quelconque des revendications précédentes, dans laquelle le composant tibial comprend un composant méniscal supporté de manière rotative sur une plate-forme tibiale.

12. Prothèse TKR selon l'une quelconque des revendications précédentes, comprenant :
des surfaces d'appui tibiales formées de sorte que lorsqu'elles sont observées comme une série de vues anatomiques transversales s'étendant de manière transversale par rapport à la direction antérieure-postérieure, les points de contact (5, 8, 9, 10) entre les surfaces d'appui sont déplacés de manière postérieure avec la flexion et de manière antérieure avec l'extension ou vice versa, et chaque point de contact (5, 8, 9, 10) constitue le point le plus bas sur la surface d'appui tibiale selon un angle de flexion donné.
